# EUROPÄISCHE PATENTSCHRIFT

(11) **EP 0 581 097 B1**
(45) Veröffentlichungstag und Bekanntmachung des Hinweises auf die Patenterteilung: **25.11.1998**
(21) Anmeldenummer: 93111114.0
(22) Anmeldetag: 12.07.1993
(51) Int. Cl.: C07C 43/303, C07C 41/50

(54) **Verfahren zur Herstellung von E,Z-Butendial-bis-dialkylacetalen**
Process for the preparation of E,Z-butenedial bis-dialkyl acetals
Procédé pour la préparation d'acétals bis-dialkyliques de butènedial-E,Z

(30) Priorität: 21.07.1992 DE 4223889
(43) Veröffentlichungstag der Anmeldung: 02.02.1994
(73) Patentinhaber: BASF Aktiengesellschaft, 67063 Ludwigshafen (DE)
(72) Erfinder: Paust, Joachim, Dr., D-6708 Neuhofen (DE); Krause, Wolfgang, Dr., D-6800 Mannheim 81 (DE)

(56) Entgegenhaltungen:
- NL-C- 67 870
- US-A- 2 879 278
- RECUEIL DES TRAVAUX CHIMIQUES DES PAYS-BAS Bd. 94, Nr. 8 , August 1975 Seiten 196 - 198 J. W. SCHEEREN ET AL 'CHEMISTRY OF ELECTRON-RICH CONJUGATED POLYENES (II). SYNTHESIS OF 1,1-DIMETHOXY- AND 1,1,4-TRIALKOXY-1,3-BUTADIENES AND OF 1,1,6,6-TETRAMETHOXY-1,3,5-HEXATRIENE'
- 'METHODEN DER ORGANISCHEN CHEMIE (HOUBEN-WEYL) BAND VI/3' 1965 , GEORG THIEME VERLAG , STUTTGART * Seite 221 - Seite 222 *

## Beschreibung

Die Erfindung betrifft ein verbessertes Verfahren zur Herstellung von E,Z-Butendial-bis-dialkylacetalen der Formel I 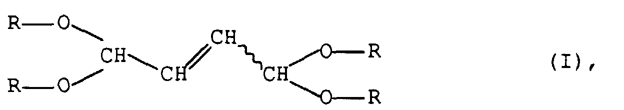 in denen R für -CH₃, -C₂H₅ oder -C₃H₇ steht, ausgehend von den entsprechenden 2,5-Dialkoxy-2,5-dihydrofuranen.

Elektronenreiche Alkene, wie die Butendial-bis-dialkylacetale, oft auch als Tetraalkoxy-2-butene bezeichnet, zeigen interessante Eigenschaften in Additions- und Cycloadditionsreaktionen mit zahlreichen elektrophilen Reaktionspartnern.

So läßt sich beispielsweise Z-Butendial-bis-diethylacetal gemäß dem Verfahren von CH 321 106 durch doppelte Enoletherkondensation mit 1-Propenyl-ethylether und anschließende Hydrolyse in guten Ausbeuten in das 2,7-Dimethylocta-2,4,6-trien-1,8-dial umsetzen. Dieser C₁₀-Dialdehyd ist ein begehrtes Zwischenprodukt für Carotinoidsynthesen (vgl. DE 28 01 908).

Die bisher bekannten Verfahren zur Herstellung der 1,1,4,4-Tetraalkoxy-2-butene sind für eine technische Herstellung nicht geeignet. So ist zum Beispiel aus GB 747,281 ein Verfahren bekannt, gemäß dem Furan unter wasserfreien Bedingungen bei Temperaturen unterhalb von -25°C mit Brom und einem Alkanol in die 1,1,4,4-Tetraalkoxy-2-butene überführt werden kann. Dieses Verfahren ist jedoch technisch und energetisch zu aufwendig und auch nicht umweltfreundlich.

Aus NL 67870 ist ein Verfahren zur Herstellung von 1,4-Dicarbonylverbindungen durch Hydrolyse von 2,5-Dialkoxy-2,5-dihydrofuranen in Gegenwart einer Säure bekannt. Es wird zwar hierin erwähnt, daß man beim Erhitzen des 2,5-Dialkoxy-2,5-dihydrofurans mit einem ortho-Ameisensäureester in Gegenwart von wenig Salzsäure Bisdialkylacetale der 1,4-Dicarbonyl-verbindungen erhalten könne. Entsprechende Versuche waren jedoch nicht erfolgversprechend.

Weiterhin wird in Recueil, J. of the Royal Netherlands Chem. Soc. 94/8 (1975) 196-98 ein Verfahren zur Herstellung von cis-1,1,4,4-Tetramethoxy-2-buten beschrieben, bei dem 2,5-Dimethoxy-2,5-dihydrofuran in Methanol mit HCOOH für 8 Stunden unter Rückfluß zum Sieden erhitzt wird. Nachteilig an diesem Verfahren ist, daß die dabei erzielten Ausbeuten trotz langer Reaktionszeiten nur etwa 45 % der Theorie betragen.

Es war daher die Aufgabe der Erfindung, ein Verfahren zur Herstellung von 1,1,4,4-Tetraalkoxy-2-butenen zu entwickeln, bei dem die Nachteile des Standes der Technik behoben werden, d.h. daß man die gewünschten Verbindungen auf einfachere und umweltschonendere Weise in guten Ausbeuten erhält.

Gegenstand der Erfindung ist dementsprechend ein Verfahren zur Herstellung von E,Z-Butendial-bis-dialkylacetalen der allgemeinen Formel I 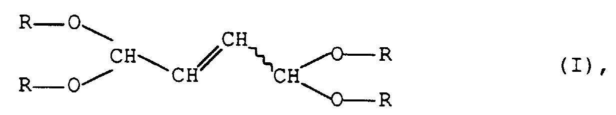 in der die Substituenten R gleich oder verschieden sein können und für -CH₃, -C₂H₅ oder -C₃H₇ stehen, durch Umsetzen des 2,5-Dialkoxy-2,5-dihydrofurans der allgemeinen Formel II 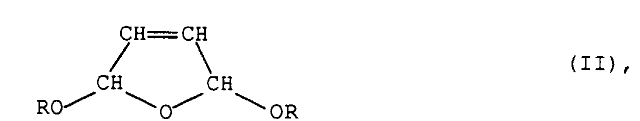 in der R für -CH₃, -C₂H₅ oder -C₃H₇ steht, mit einem Alkanol der Formel III
R OH (III), in der R für -CH₃, -C₂H₅ oder -C₃H₇ steht, in Gegenwart einer Säure bei erhöhter Temperatur, das dadurch gekennzeichnet ist daß man die Umsetzung in Gegenwart etwa äquimolarer Mengen, d.h. etwa 0,9 bis 1,5 Mol pro Mol des Furans der allgemeinen Formel II, eines Ameisensäure-ortho-alkylesters der Formel IV

HC(OR)₃ (IV),

in der R für -CH₃, -C₂H₅ oder C₃H₇ steht, und in Gegenwart katalytischer Mengen einer Mineralsäure oder einer starken organischen Säure, die die Reaktionspartner nicht anderweitig angreifen, als Katalysator, durchführt.

Mit besonderem Vorteil gelingt das erfindungsgemäße Verfahren, wenn man es in Gegenwart einer organischen Sulfonsäure, insbesondere in Gegenwart von p-Toluolsulfonsäure als Katalysator durchführt.

Die als Ausgangsverbindungen benötigten 2,5-Dialkoxy-2,5-dihydrofurane der Formel II sind als Zwischenprodukte zur Herstellung des bioziden Succinaldehyds industriell gut verfügbar und werden technisch auf einfache Weise beispielsweise dadurch zugänglich, daß man Furan elektrochemisch in Alkanolen oxidiert.

Als Ameisensäure-ortho-alkylester der Formel IV nimmt man mit Vorteil Ameisensäure-ortho-methylester, man kann aber auch Ameisensäure-ortho-ethylester oder gar Ameisensäureortho-propylester verwenden.

Der Ameisensäureester dient als wasserabspaltendes Mittel und wird daher in etwa molaren Mengen eingesetzt, d.h. in Mengen von etwa 0,9 bis 1,5, vorzugsweise 1,0 bis 1,2 Mol pro Mol des Furans der allgemeinen Formel II.

Als Alkanol der Formel III verwendet man vorzugsweise dasjenige Alkanol, das den gleichen Alkylrest trägt wie das eingesetzte Furan der Formel II. Man kann zwar auch beliebige Alkanole einsetzen, erhält dann aber kein einheitliches Produkt, sondern ein Gemisch aus Butendial-bis-dialkyl-acetalen mit verschiedenen Alkylsubstituenten in einem Molekül.

Mit besonderem Vorteil gestaltet sich das erfindungsgemäße Verfahren, wenn man zur Herstellung von E,Z-Butendial-bis-dimethylacetal der Formel I 2,5-Dimethoxy-2,5-dihydrofuran der Formel II in Gegenwart von Ameisensäure-ortho-methylester mit Methanol umsetzt.

Das Alkanol der Formel III verwendet man im allgemeinen in Mengen von 2 bis 10, vorzugsweise 3 bis 6 Mol Alkanol pro Mol des Furans der Formel II.

Als für das erfindungsgemäße Verfahren geeignete Katalysatoren werden Mineralsäuren oder organische Säuren mit einem Säureexponenten (pKs-Wert) von etwa -9 bis +3,77 verstanden, die die Reaktionspartner unter den Reaktionsbedingungen nicht anderweitig angreifen. Bezüglich der Definition des Säureexponenten bzw. des pKs-Wertes verweisen wir auf Ullmanns Encyklopädie der technischen Chemie, Band 15, Seite 2. Geeignet sind Mineralsäuren, wie beispielsweise konzentrierte Schwefelsäure (pKs = -3), zweckmäßig 90 bis 98 gew.-%ige Schwefelsäure, ortho-Phosphorsäure (pKs = 2,09), zweckmäßig 85 bis 90 gew.-%ige Phosphorsäure, konz. Salzsäure (pKs = -6), Perchlorsäure (pKs = -9) sowie starke organische Säuren, wie Trichloressigsäure (pKs = 0,92), Ameisensäure (pKs-Wert = 3,77), Oxalsäure (pKs = 1,46), und organische Sulfonsäuren. Als geeignete Sulfonsäuren seien genannt: aliphatische Sulfonsäuren, insbesondere die niederen Alkansulfonsäuren, wie Methansulfonsäure, Ethansulfonsäure, 1-Propansulfonsäure, 2-Propan-sulfonsäure und Trifluormethansulfonsäure; die alicyclischen Sulfonsäuren wie Camphersulfonsäure, und die aromatischen Sulfonsäuren wie die Toluolsulfonsäuren, insbesondere die p-Toluolsulfonsäure, die Naphthalinsulfonsäuren, insbesondere Naphthyl-1-sulfonsäure und Naphthyl-2-sulfonsäure, Benzolsulfonsäure sowie Sulfonsäuregruppen enthaltende saure Ionenaustauscher. Von den erwähnten starken Säuren werden in dem erfindungsgemäßen Verfahren insbesondere die relativ billigen, starken, anorganischen Säuren wie Schwefelsäure und Phosphorsäure, sowie die relativ wenig korrosiven, starken organischen Säuren wie p-Toluolsulfonsäure bevorzugt.

Den sauren Katalysator verwendet man im allgemeinen in Mengen von etwa 10⁻⁵ bis 5·10⁻², vorzugsweise 10⁻⁴ bis 10⁻² Mol, pro Mol 2,5-Dimethoxy-2,5-dihydrofuran im Falle der anorganischen Säure bzw. 10⁻⁵ bis 10⁻¹, vorzugsweise 10⁻⁴ bis 10⁻² Mol im Falle der organischen Säure.

Zur Durchführung der Reaktion geht man im allgemeinen so vor, daß man eine Lösung der Reaktionspartner I bis IV mit dem sauren Katalysator versetzt und das Reaktionsgemisch unter Rückfluß zum Sieden erhitzt.

Die Reaktionszeigt beträgt im allgemeinen 1 bis 10, vorzugsweise 2 bis 6 Stunden. Den Fortgang der Reaktion kann man durch gaschromatographische Analyse verfolgen.

Mit Hilfe des erfindungsgemäßen Verfahrens können die als Zwischenprodukte für Carotinoidsynthesen begehrten E,Z-Butendial-bis-dialkylacetale der allgemeinen Formel I auf einfache Weise in guten Ausbeuten erhalten werden.

### Beispiel 1

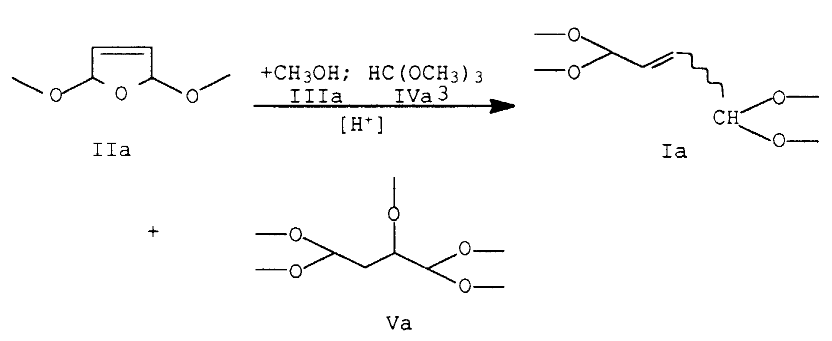

Eine Lösung von 265,3 g (2 mol) 2,5-Dimethoxy-2,5-dihydrofuran (IIa), 384 g (12 mol) Methanol (IIIa) und 214,4 g (2 mol) ortho-Ameisensäuretrimethylester (IVa) wurde in einem 2 1-Glasreaktor mit 0,2 g p-Toluolsulfonsäure versetzt, dann in 20 Minuten (min) auf 65°C erwärmt und schließlich für 2 Stunden (h) unter Rückfluß zum Sieden erhitzt. Die Umsetzung wurde gaschromatographisch (Flächenprozente) verfolgt. Die folgende Tabelle zeigt den Reaktionsverlauf unter Bildung von E,Z-Butendial-bis-dimethylacetal (Ia) und 1,1,2,4,4-Pentamethoxy-butan (Va).

| Reaktionszeit [min] | HC(OCH₃)₃ [Fl.-%] | IIa [Fl.-%] | Ia [Fl.-%] | Va [Fl.-%] |
|---|---|---|---|---|
| 0 | 29,5 | 59 | 9,4 | |
| 30 | 18,2 | 33 | 47 | 0,6 |
| 60 | 14,0 | 26 | 57 | 1,0 |
| 90 | 11,0 | 21 | 65 | 1,8 |
| 120 | 7,7 | 15 | 72 | 2,9 |

Anschließend stoppte man die Umsetzung durch Zugabe von 1,9 g einer 30 %igen Lösung von CH₃ONa in Methanol, destillierte überschüssiges Methanol bei Normaldruck über eine Brücke ab und fraktionierte den Rückstand durch Destillation unter stark vermindertem Druck in einer 30 cm Füllkörperkolonne. Die erhaltenen Fraktionen hatten nach gaschromatographischer Analyse (GC) folgende Zusammensetzung:

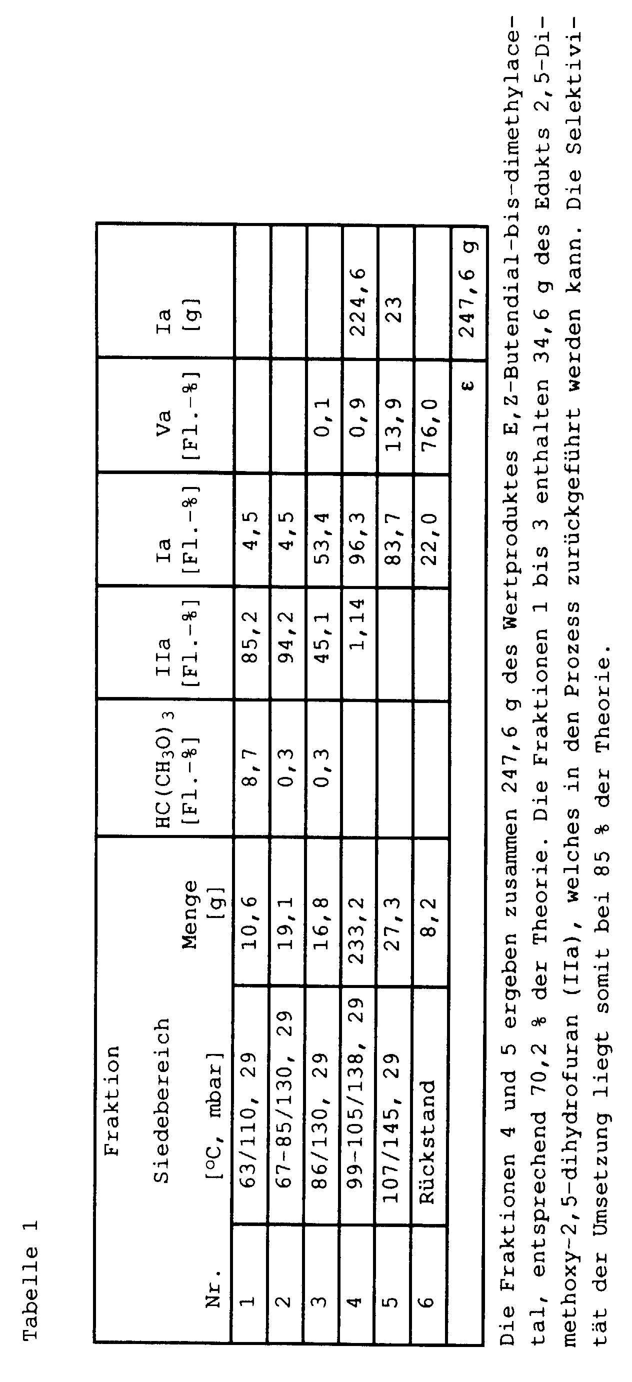

### Beispiel 2

Eine Lösung von 198 g (1,5 mol) 2,5-Dimethoxy-2,5-dihydrofuran in einem Gemisch aus 288 g (9 mol) Methanol und 191 g (1,8 mol) ortho-Ameisensäuretrimethylester wurde mit 0,1 g konz. H2S04 versetzt. Danach wurde das Reaktionsgemisch 4 h unter Rückfluß (69°C) zum Sieden erhitzt. Die Reaktion wurde durch Zusatz von 1,5 g einer 30 %igen Lösung von CH3ONa in Methanol abgebrochen. Anschließend wurden bei Normaldruck Methanol und Methylformiat abdestilliert. Der verbleibende Rückstand wurde bei ca. 30 mbar fraktionierend destilliert (96 bis 103°C). Es wurden 198 g eines 98 %igen Butendialbisdimethylacetals erhalten, entsprechend einer Ausbeute von 74 % der Theorie. Nicht umgesetztes 2,5-Dimethoxy-2,5-dihydrofuran wurde als Vorlauf isoliert und kann erneut in die Umsetzung eingesetzt werden.

### Beispiele 3 bis 9

Jeweils die aus der Tabelle 2 ersichtliche Menge an 2,5-Dimethoxy-2,5-dihydrofuran (IIa), Methanol, ortho-Ameisensäuretrimethylester und saurer Katalysator wurden zusammen in einem 2 1 Glasreaktor zunächst in 20 min auf 65°C erwärmt und dann für die in der Tabelle angegebene Reaktionszeit unter Rückfluß zum Sieden erhitzt. Die jeweils erzielten Ausbeuten an E,Z-Butendial-bis-dimethylacetal sind in der letzten Spalte von Tabelle 2 angegeben. Da die Umsetzung mit einem Umsatz von etwa 90 % verläuft, ergeben sich somit Selektivitäten von etwa 80 bis 85 % der Theorie.

**Tabelle 2**

| Beispiel | IIa [mol] | HC(CH₃O)₃ [mol] | CH₃OH [mol] | saurer Katalysator [g] | Reaktionszeit [h] | Ausbeute [%] |
|---|---|---|---|---|---|---|
| 3 | 2 | 2 | 6 | 0,1 g konz. H₂SO₄ | 4 | 74-78 |
| 4 | 2 | 2 | 9 | 4 g konz. HCl | 5 | 72 |
| 5 | 2 | 2 | 6 | 1 g konz. H₃PO₄ | 6 | 72 |
| 6 | 2 | 2 | 6-12 | 0,2 g p-Tos-OH | 2 | 75 |
| 7 | 2 | 2 | 6 | 0,2 g CF₃-SO₃H | 2 | 74 |
| 8 | 2 | 2 | 6 | 0,2 g CH₃-SO₃H | 2 | 73 |
| 9 | 2 | 2 | 6-12 | 1 g "Sicapent" (P₂O₅ auf SiO₂) | 6 | 70 |

### Vergleichsbeispiel

Eine Lösung von 265,3 g (2 mol) 2,5-Dimethoxy-2,5-dihydrofuran in 192 g (6 mol) Methanol wurde mit 0,2 g p-Toluolsulfonsäure versetzt und danach 1 h unter Rückfluß zum Sieden erhitzt. Anschließend wurde innerhalb von 1,5 h über eine 10 cm Vigreux-Kolonne Methanol abdestilliert und während dieser Destillation erneut 6 Mol Methanol langsam zugegeben (Wasserauskreisen). Nach einer Reaktionszeit von insgesamt 8 h wurde das Methanol vollständig abdestilliert, der Rückstand über die beschriebene Vigreux-Kolonne destillativ getrennt. Hierbei wurden 60 % des eingesetzten 2,5-Dimethoxy-2,5-dihydrofurans unumgesetzt zurückerhalten. Die Ausbeute an dem gewünschten Butendialbisdimethylacetal betrug nur ca. 35 % der Theorie.

## Patentansprüche

1. Verbessertes Verfahren zur Herstellung von E,Z-Butendial-bis-dialkylacetalen der allgemeinen Formel I 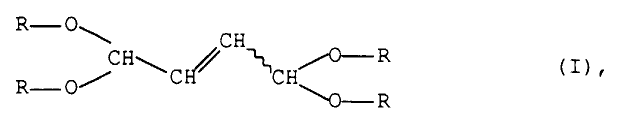 in der die Substituenten R gleich oder verschieden sein können und für -CH₃, -C₂H₅ oder -C₃H₇ stehen, durch Umsetzen des 2,5-Dialkoxy-2,5-dihydrofurans der allgemeinen Formel II 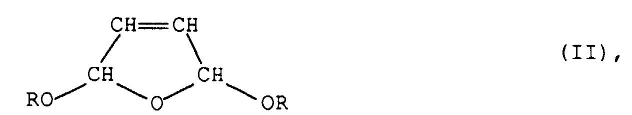 in der R für -CH₃, -C₂H₅ oder -C₃H₇ steht, mit einem Alkanol der Formel III
R OH (III), in der R für -CH₃, -C₂H₅ oder -C₃H₇ steht,
in Gegenwart einer Säure bei erhöhter Temperatur, das dadurch gekennzeichnet ist, daß man die Umsetzung in Gegenwart von 0,9 bis 1,5 Mol eines Ameisensäure-ortho-alkylesters der Formel IV
HC(OR)₃ (IV),
in der R für -CH3, -C₂H₅ oder -C₃H₇ steht, pro Mol des Furans der allgemeinen Formel II und in Gegenwart katalytischer Mengen einer Mineralsäure oder einer starken organischen Säure, die die Reaktionspartner nicht anderweitig angreifen, als Katalysator, durchführt.

2. Verfahren gemäß Anspruch 1, dadurch gekennzeichnet, daß man zur Herstellung von E,Z-Butendial-bis-dimethylacetal der Formel I 2,5-Dimethoxy-2,5-dihydrofuran der Formel II in Gegenwart von Ameisensäure-ortho-methylester mit Methanol umsetzt.

3. Verfahren gemäß Anspruch 1, dadurch gekennzeichnet, daß man die Umsetzung in Gegenwart einer organischen Sulfonsäure als Katalysator durchführt.

4. Verfahren gemäß Anspruch 3, dadurch gekennzeichnet, daß man die Umsetzung in Gegenwart von p-Toluolsulfonsäure als Katalysator durchführt.

## Claims

1. An improved process for preparing E,Z-butenedial bis(dialkyl acetals) of the formula I 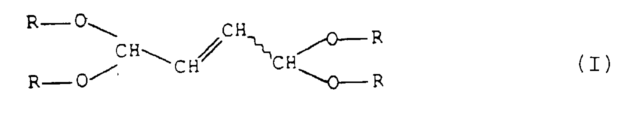 where the substituents R can be identical or different and are -CH₃, -C₂H₅ or -C₃H₇, by reacting the 2,5-dialkoxy-2,5-dihydrofuran of the formula II 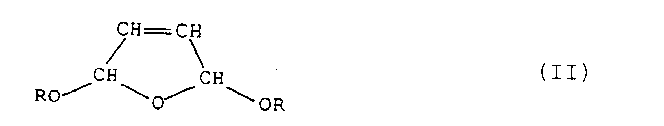 where R is -CH₃, -C₂H₅ or -C₃H₇, with an alkanol of the formula III
R OH (III) where R is -CH₃, -C₂H₅ or -C₃H₇, in the presence of an acid at elevated temperature, wherein the reaction is carried out in the presence of from 0.9 to 1.5 mol of a trialkyl orthoformate of the formula IV
HC(OR)₃ (IV)
where R is -CH₃, -C₂H₅ or -C₃H₇, per mole of the furan of the formula II and in the presence of catalytic amounts of a mineral acid or of a strong organic acid, neither of which attacks the reactants in another way, as catalyst.

2. A process as claimed in claim 1, wherein 2,5-dimethoxy-2,5-dihydrofuran of the formula II is reacted with methanol in the presence of trimethyl orthoformate to prepare E,Z-butenedial bis(dimethyl acetal) of the formula I.

3. A process as claimed in claim 1, wherein the reaction is carried out in the presence of an organic sulfonic acid as catalyst.

4. A process as claimed in claim 3, wherein the reaction is carried out in the presence of p-toluenesulfonic acid as catalyst.

## Revendications

1. Procédé perfectionné pour la préparation des bis-dialkylacétals du E,Z-butènedial de formule générale I 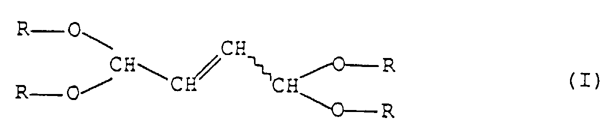 dans laquelle les symboles R, ayant des significations identiques ou différentes, représentent chacun -CH₃, -C₂H₅ ou -C₃H₇, par réaction d'un 2,5-dialcoxy-2,5-dihydrofuranne de formule générale II 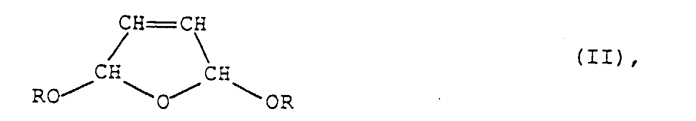 dans laquelle R représente -CH₃, -C₂H₅ ou -C₃H₇, avec un alcanol de formule III
R OH (III),
dans laquelle R représente -CH₃, -C₂H₅ ou -C₃H₇,
en présence d'un acide et à chaud, ce procédé se caractérisant par le fait que l'on exécute la réaction en présence de 0,9 à 1,5 mol d'un ester ortho-alkylique de l'acide formique de formule IV
HC(OR)₃ (IV),
dans laquelle R représente -CH₃, -C₂H₅ ou -C₃H₇, pour une mole du furanne de formule générale II, et en présence de quantités catalytiques d'un acide minéral ou d'un acide organique fort inerte par ailleurs à l'égard des composants de la réaction et qui sert de catalyseur.

2. Procédé selon la revendication 1, caractérisé par le fait que, pour préparer le bis-diméthylacétal du E/Z-butène-dial de formule I, on fait réagir le 2,5-diméthoxy-2,5-dihydrofuranne de formule II avec le méthanol en présence de l'ester orthométhylique de l'acide formique.

3. Procédé selon la revendication 1, caractérisé par le fait que la réaction est effectuée en présence d'un acide sulfonique organique qui sert de catalyseur.

4. Procédé selon la revendication 3, caractérisé par le fait que la réaction est effectuée en présence d'acide p-toluènesulfonique qui sert de catalyseur.
